# EUROPEAN PATENT APPLICATION

(11) **EP 4 321 137 A2**
(43) Date of publication of application: **14.02.2024**
(21) Application number: 23211246.6
(22) Date of filing: 13.05.2020
(51) Int. Cl.: A61F 13/15

(54) **DISPOSABLE DIAPER FOR PET AND PACKAGE OF THE DISPOSABLE DIAPERS FOR PET**

(30) Priority: 20.05.2019 JP 2019094870
(62) Divisional of application: 20174315.0
(71) Applicant: Unicharm Corporation, Shikokuchuo-shi, Ehime 799-0111 (JP)
(72) Inventor: KOMATSUBARA, Daisuke, Kanonji-shi, 769-1602 (JP)
(74) Representative: Staeger & Sperling Partnerschaftsgesellschaft mbB

(57) **Abstract**

The disclosure relates to a disposable diaper for pet (1), comprising a waistline direction (W) disposed along a waistline direction of a pet; a cross direction (Z) orthogonal to the waistline direction (W); a main body portion (2) having a topsheet (10), a backsheet (20), and an absorbent core (30) arranged between the topsheet (10) and the backsheet (20); a binding portion (40, 40X) that extends outwards in the waistline direction (W) from the main body portion (2), in a main-body third end (63) that resides on one end side in the cross direction (Z) of the main body portion (2); and a target part (45) with which the binding portion (40, 40X) is bindable, and the disposable diaper for pet (1) being intended to be placed to cover the pet from a belly through a crotch to a back of the pet, characterized in that the disposable diaper for pet (1) has a plurality of mark portions (50) having mark portions (55, 56, 57) in the cross direction (Z) and mark portions (58) in the waistline direction (W), the mark portions (55, 56, 57) in the cross direction (Z) are arranged with an interval therebetween in the cross direction (Z), the mark portions (58) in the waistline direction (W) are arranged with an interval therebetween in the waistline direction (W), and the mark portions (58) in the waistline direction (W) are arranged in the target part (45).

## Description

### TECHNICAL FIELD

The present invention relates to a disposable diaper for pet and a package for disposable diapers for pet.

### BACKGROUND ART

Patent Literatures 1 and 2 disclose disposable diapers for pet. The disposable diaper for pet disclosed in Patent Literature 1 has a belly pad to be placed on the ventral side of a pet, a back pad to be placed on the dorsal side of the pet, a tail hole allowed for insertion of a tail through the back pad, and a fastener tape arranged to the belly pad. When the disposable diaper for pet disclosed in Patent Literature 1 is placed, the belly pad is placed on the ventral side of the pet, the back pad is placed on the dorsal side, while inserting the tail through the tail hole, and the fastener tape is attached on the back pad (see Fig. 4 of Patent Literature 1).

The disposable diaper for pet disclosed in Patent Literature 2 has a band-like main body having an absorbent core, an adhesive seal arranged on the top face side of the main body, and a seal receiving part arranged on the back face side of the main body. When the disposable diaper for pet disclosed in Patent Literature 2 is placed, the disposable diaper for pet is positioned so as to align the longitudinal direction of the main body to the waistline direction of a pet, and to cover the urethral opening of the pet with the absorbent core, thereby wrapping around the waist of the pet with the aid of the adhesive seal and the seal receiving part (see Fig. 3 of Patent Literature 2).

### CITATION LIST

### PATENT LITERATURE

Patent Literature 1: JP 2003-210062 A
Patent Literature 2: JP 3141580 U
Further prior art in this technical field is disclosed in documents EP 2 721 924 A1, EP 3 476 213 A1 and EP 2 932 834 A1

### SUMMARY OF INVENTION

Ordinary disposable diapers for pet are available in various sizes, and the user chooses a suitable size depending on species or degrees of growth of its pet. Some users, however, could not know how to choose the size, and would sometimes fail in using a disposable diaper for pet with a suitable size.

Meanwhile, the user can feel growth of the pet, through changes of suitable size of disposable diaper. Some user even enjoys the degree of growth of the pet, through sensing fitting quality of the disposable diaper or changes of size.

It is therefore an object of the present invention to provide a disposable diaper for pet, capable of making the user easily recognize the degree of growth of its pet upon placement of the disposable diaper.

A disposable diaper for pet according to one embodiment of the disclosure includes : a waistline direction disposed along a waistline direction of a pet; a cross direction orthogonal to the waistline direction; a main body portion having a topsheet, a backsheet, and an absorbent core arranged between the topsheet and the backsheet; and a binding portion arranged in a main-body first end which resides on one end side in the waistline direction of the main body portion. The disposable diaper for pet being intended to be placed on a pet so as to wrap a waist of the pet. The disposable diaper for pet having a plurality of mark portions arranged with an interval therebetween in the waistline direction.

A disposable diaper for pet according the invention comprises the features of claim 1 and includes: a waistline direction disposed along a waistline direction of a pet; a cross direction orthogonal to the waistline direction; a main body portion having a topsheet, a backsheet, and an absorbent core arranged between the topsheet and the backsheet; and a binding portion arranged in a main-body first end which resides on one end side in the waistline direction of the main body portion. The disposable diaper for pet being intended to be placed on a pet so as to wrap a waist of the pet. The disposable diaper for pet having a plurality of mark portions arranged with an interval therebetween in the cross direction.

A package of a disposable diaper for pet according to one embodiment includes a plurality of disposable diapers for pet , and a wrapper that encloses the plurality of disposable diapers for pet. The disposable diapers for pet include a first disposable diaper having a first decoration portion and the mark portion printed on the backsheet, and a second disposable diaper having a second decoration portion designed differently from the first decoration portion, and the mark portion printed on the backsheet. The backsheet is arranged over the entire range of the main body portion in a continuous direction that contains at least either the waistline direction or the cross direction.

A method for manufacturing disposable diaper for pet according to the disclosure includes a sheet feeding step of feeding a continuous sheet having sheet components arrayed; a marking step of giving a mark portion on the continuous sheet; and a core laminating step of laminating an absorbent core on the continuous sheet having the mark portion. The core laminating step being designed to detect a position of the mark portion on the continuous sheet, and to adjust a position of lamination of the absorbent core referring to the position of the mark portion.

### BRIEF DESCRIPTION OF DRAWINGS

[Fig. 1] Fig. 1 is a plan view in which a disposable diaper according to a first embodiment of the present invention is seen from a skin contact surface side.
[Fig. 2] Fig. 2 is a plan view illustrating the disposable diaper for pet of the first embodiment, when viewed from the non-skin contact side.
[Fig. 3] Fig. 3 is a schematic cross sectional view of the disposable diaper for pet taken along the line A-Ain Fig. 1.
[Fig. 4] Fig. 4 is a drawing schematically illustrating a wearing state of the disposable diaper for pet.
[Fig. 5] Fig. 5 is a plan view illustrating the disposable diaper for pet of the second embodiment, when viewed from the skin contact side.
[Fig. 6] Fig. 6 is a plan view illustrating the disposable diaper for pet of the second embodiment, when viewed from the non-skin contact side.
[Fig. 7] Fig. 7 is a drawing schematically illustrating a wearing state of the disposable diaper for pet of the second embodiment.
[Fig. 8] Fig. 8 is a drawing illustrating a package of disposable diapers for pet according to one embodiment.
[Fig. 9] Fig. 9 is a drawing illustrating a package of disposable diapers for pet according to modified example.

### (1) Outline of Embodiment

According to the present specification and the accompanying drawings, at least the following matters will be disclosed. Thereby embodiments not falling under the scope of claim 1 or claim 4 are not part of the invention.

A disposable diaper for pet according to one embodiment includes : a waistline direction disposed along a waistline direction of a pet; a cross direction orthogonal to the waistline direction; a main body portion having a topsheet, a backsheet, and an absorbent core arranged between the topsheet and the backsheet; and a binding portion arranged in a main-body first end which resides on one end side in the waistline direction of the main body portion. The disposable diaper for pet being intended to be placed on a pet so as to wrap a waist of the pet. The disposable diaper for pet having a plurality of mark portions arranged with an interval therebetween in the waistline direction. According to this embodiment, the user can recognize the degree of growth in the waistline direction of pet, referring to the mark portions. With the mark portions arranged with an interval therebetween in the waistline direction., the user can recognize the degree of growth, such as increase of the waist size or arrival at the upper limit of suitable size, referring to a positional relation between an arbitrary point of the disposable diaper and the mark portions in the wearing state. The user can therefore choose a suitable size, referring to the mark portions.

According to a embodiment, the plurality of mark portions is also arranged with an interval therebetween in the cross direction. The cross direction of the disposable diaper for pet, intended to be placed on a pet so as to wrap the waist, extends from the crotch side to a side apart from the crotch. With the plurality of mark portions arranged in the cross direction, the user can recognize changes of the waist size in the direction from the crotch side to the side apart from the crotch. For example, the user can recognize, from bulge or dent of the belly, not only the growth process but also changes in physical condition.

According to a embodiment, the binding portion is arranged on a face on a back face side of the main-body first end, and the mark portion is arranged so as to be visually recognizable from the back face side of the main-body first end. When the binding portion arranged on a face on the back face side of the main-body first end is bound with the main-body second end, the main-body second end will reside outside of the main-body first end. The user, in the process of placement, visually recognizes the edge of the main-body second end from the outer face (back face) side. The user in this process can recognize the waist size referring to the positional relation between a position of the edge of the main-body second end and the mark portions, and can confirm suitability of size or can enjoy the growth process.

According to a embodiment, the binding portion is arranged on a face on the back face side of the main-body first end, and the mark portion is arranged so as to be visually recognizable from a top face side of a main-body second end that resides on the other end side in the waistline direction of the main body portion. When the binding portion arranged on the face on the back face side of the main-body first end is bound with the main-body second end, the main-body second end will reside outside of the main-body first end, and the back face of the main-body first end and the top face of the main-body second end come into contact. The user can visually recognize, in the process of placement, the mark portions and the binding portion that are brought into contact. The user can recognize the waist size referring to the positional relation between the mark portions of the main-body second end and the binding portion, and can confirm suitability of size or can enjoy the growth process.

According to a embodiment, the binding portion is arranged on a face on the top face side of the main-body first end, and the mark portion is arranged so as to be visually recognizable from a back face side of the main-body second end that resides on the other end side in the waistline direction of the main body portion. When the binding portion arranged on a face on the top face side of the main-body first end is bound with the main-body second end, the main-body first end will reside outside of the main-body second end. The user, in the process of placement, visually recognizes the outer edge of the main-body first end from the outer face (back face) side. In this process, the user can recognize the waist size referring to the positional relation between a position of the edge of the main-body first end and the mark portions, and can confirm suitability of size or can enjoy the growth process.

A disposable diaper for pet according to another embodiment includes : a waistline direction disposed along a waistline direction of a pet; a cross direction orthogonal to the waistline direction; a main body portion having a topsheet, a backsheet, and an absorbent core arranged between the topsheet and the backsheet; and a binding portion arranged in a main-body first end which resides on one end side in the waistline direction of the main body portion. The disposable diaper for pet being intended to be placed on a pet so as to wrap a waist of the pet. The disposable diaper for pet having a plurality of mark portions arranged with an interval therebetween in the cross direction. The user can recognize the degree of growth in the longitudinal direction that extends from the belly through the crotch to the back of the pet referring to the mark portions. With the mark portions arranged with an interval therebetween in the cross direction, the user can recognize the degree of growth, such as increase of the longitudinal size or arrival at the upper limit of suitable size, referring to a positional relation between an arbitrary point of the disposable diaper and the mark portions in the wearing state. The user can therefore choose a suitable size, referring to the mark portions.

According to a preferred embodiment, the main body portion has a pair of leg surrounding openings to be arranged around legs of pet, composed of outer edges in the waistline direction of the main body portion recessed inwards in the waistline direction. At least one mark portion, out from the plurality of mark portions, is arranged in a region that falls between the pair of leg surrounding openings. When the disposable diaper is placed so as to cover the pet from the belly through the crotch to the back of the pet, the binding portion is preferably bound along a line which extends from the base of the legs on the ventral side to the dorsal side. With the binding portion bound along the line which extends from the base of legs on the ventral side to the dorsal side, fitting quality around the legs will improve, and thereby leakage or displacement may be suppressed. With the mark portions arranged in a region that falls between the leg surrounding openings, the binding portion may be bound referring to the mark portions in a region around the legs.

According to a preferred embodiment, the plurality of mark portions is also arranged with an interval therebetween in the waistline direction. With the mark portions in this embodiment arranged with an interval therebetween in the waistline direction., the user can recognize the degree of growth, such as increase of the waist size or arrival at the upper limit of suitable size, referring to a positional relation between an arbitrary point of the disposable diaper and the mark portions in the wearing state. The user can therefore choose a suitable size, referring to the mark portions.

According to a preferred embodiment, the disposable diaper includes a tail hole that has a through-hole through which the tail of pet can be inserted, and a cut part that can enlarge the through-hole. The mark portion has a tail mark portion that is formed so as to be visually recognizable from the backsheet side of the disposable diaper for pet, and indicates the tail hole. The user also can recognize the growth process of pet, through the thickness and length of the tail inserted through the tail hole. With the disposable diaper placed on the pet, the user can visually recognize both of the mark portions and the conditions of the tail inserted through the tail hole, and can determine whether or not to enlarge the tail hole using the cut.

A package of a disposable diaper for pet according to one embodiment includes a plurality of disposable diapers for pet , and a wrapper that encloses the plurality of disposable diapers for pet. The disposable diapers for pet include a first disposable diaper having a first decoration portion and the mark portion printed on the backsheet, and a second disposable diaper having a second decoration portion designed differently from the first decoration portion, and the mark portion printed on the backsheet. The backsheet is arranged over the entire range of the main body portion in a continuous direction that contains at least either the waistline direction or the cross direction. With the first disposable diaper having the first decoration portion and the second disposable diaper having the second decoration portion, the decorativeness can be improved. Such package can attract greater attention of the user, as compared with an embodiment in which all disposable diapers have a uniform design. The first decoration portion, the second decoration portion and the mark portions are arranged likewise on the backsheet, thereby the first decoration portion and the second decoration portion can attract attention of the user, to enhance recognizability of the mark portions.

According to a preferred embodiment, the backsheet has an outer region that extends inwards from an outer edge in the continuous direction of the backsheet, and an inner region that resides inside, in the continuous direction, of the outer region. The first decoration portion and the second decoration portion are arranged in the inner region. The first disposable diaper and the second disposable diaper have a common decoration portion having a unified design, printed in the outer regions of the first disposable diaper and the second disposable diaper. The common decoration portion is designed differently from the first decoration portion and the second decoration portion. Since each of the first disposable diaper and the second disposable diaper in this embodiment has the outer region and the inner region which are differently designed, so that the design makes the user more easily recognize the position in the main body portion. When recognizing the position of the mark portions, the user can easily find the position over the entire range of the main body portion, and can more properly understand the degree of growth of pet, referring to the mark portions. The differently designed first decoration portion and the second decoration portion can enhance the decorativeness, meanwhile the common decoration portion can make the user more easily recognize the position of components (main body portion and mark portions) over the entire range of the disposable diaper.

According to a preferred embodiment, the backsheet has an outer region that extends inwards from the outer edge in the continuous direction of the backsheet, and an inner region that resides inside, in the continuous direction, of the outer region. The first decoration portion and the second decoration portion are arranged in the inner region. Each of the first disposable diaper and the second disposable diaper has, in the outer regions of the first disposable diaper and the second disposable diaper, a non-printed region free of printing. With the non-printed part arranged in the outer region, and with the decoration portion arranged in the inner region, the user will easily recognize the position in the main body portion, referring to the design, or presence or absence of such design. When recognizing the position of the mark portions, the user can easily find the position over the entire range of the main body portion, and can more properly understand the degree of growth of pet, referring to the mark portions. The differently designed first decoration portion and the second decoration portion can enhance the decorativeness, meanwhile the common non-printed part can make the user more easily recognize the position of components (main body portion and mark portions) over the entire range of the disposable diaper.

A method for manufacturing disposable diaper for pet according to one embodiment includes a sheet feeding step of feeding a continuous sheet having sheet components arrayed; a marking step of giving a mark portion on the continuous sheet; and a core laminating step of laminating an absorbent core on the continuous sheet having the mark portion. The core laminating step being designed to detect a position of the mark portion on the continuous sheet, and to adjust a position of lamination of the absorbent core referring to the position of the mark portion. With present method for manufacturing, the positional relation between the mark portion and the absorbent core may be kept constant. Hence, the disposable diaper when placed referring to the mark portion will have the absorbent core whose placement position is prevented from being misaligned one by one. With the mark portion 50 suppressed from shifting over the whole range of the disposable diaper, it now becomes possible to improve accuracy of alignment and accuracy of pointing of the degree of growth, with the aid of the mark portions 50.

### (2) Overall Structure of Disposable diaper for pet

A disposable diaper for pet according to an embodiment will be described below by referring to the accompanying drawings. In the drawings, the same or similar parts are indicated by the same or similar reference signs. The drawings are illustrated schematically, and dimensional ratio and other variables differ from those of actual measurements. The actual measurements or the like, therefore, should be determined by referring to the following description. The drawings may include different relationships or ratios of measurements.

In this patent specification, "pet" widely encompasses vertebrate and invertebrate animals, and typically include pet animals such as cat, dog, rabbit and hamster. The disposable diaper in this embodiment is a disposable diaper for dog. In a modified example, the disposable diaper may be the one for cat.

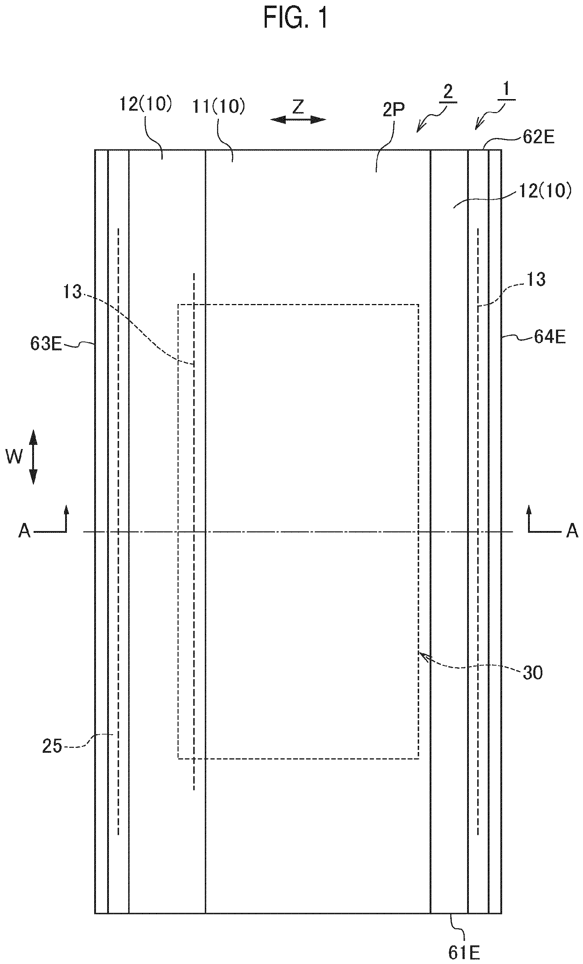

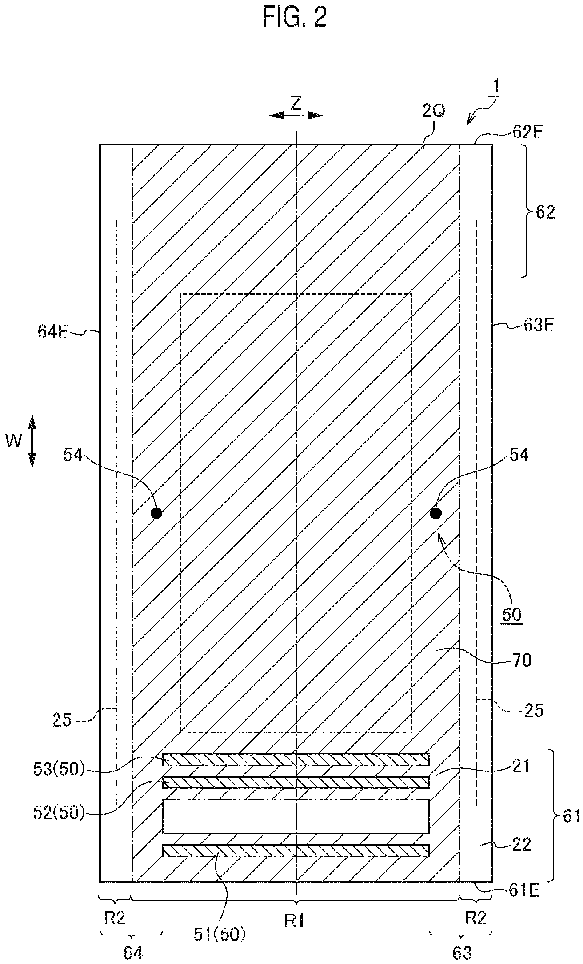

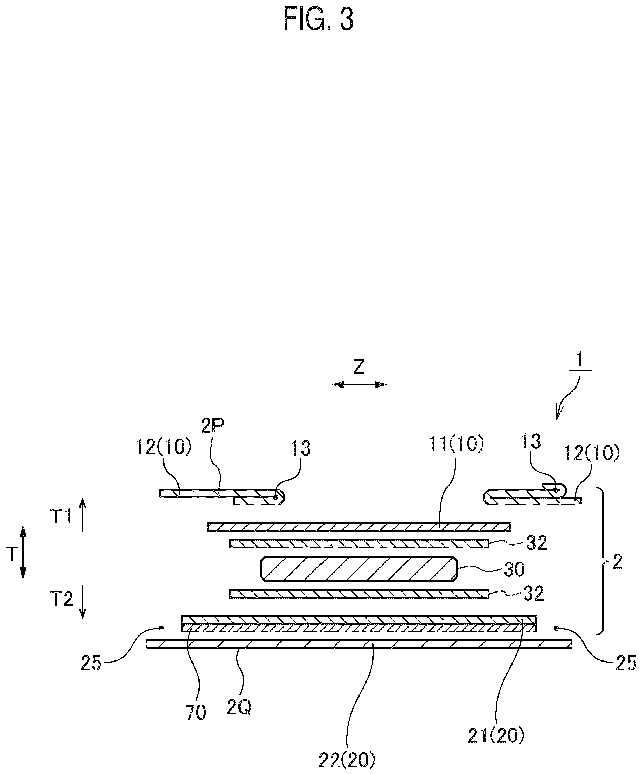

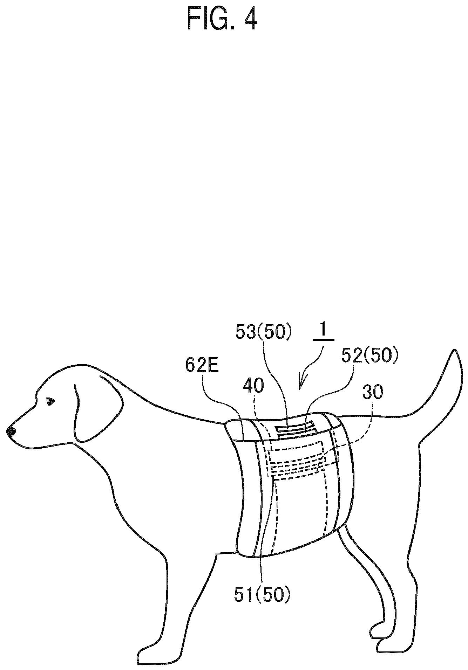

Fig. 1 is a plan view illustrating the disposable diaper for pet of the first embodiment, when viewed from the skin contact side. Fig. 2 is a plan view illustrating the disposable diaper for pet of the first embodiment, when viewed from the non-skin contact side. Fig. 3 is a schematic cross sectional view of the disposable diaper for pet taken along the line A-A in Fig. 1. Figs. 1 and 2 illustrate a disposable diaper for pet 1, extended until any wrinkle will disappear. The individual members, although illustrated in the cross sectional view of Fig. 3 as if they are apart from each other in the thickness direction T for the convenience of explanation, are brought into close contact in the thickness direction T in the actual product. Fig. 4 is a drawing schematically illustrating a wearing state of the disposable diaper for pet.

The disposable diaper for pet has waistline direction W laid in the waistline direction of the pet, cross direction Z laid orthogonal to the waistline direction W, and the thickness direction T laid orthogonal to the waistline direction W and to the cross direction Z. The cross direction Z is laid in the longitudinal direction of the pet, that is, the direction connecting the crotch-side with the far side of the crotch. The thickness direction T is laid towards the top face side T1 faced to the pet in the wearing state, and towards the back face side T2 faced outside in the wearing state. As illustrated in Fig. 4, the disposable diaper for pet of the first embodiment is placed so as to wrap around the waist of the pet, rather than being placed so as to cover the pet from the belly through the crotch to the back. Hence the length in the waistline direction W of the disposable diaper for pet 1 is longer than the length in the cross direction Z of the disposable diaper for pet 1.

The disposable diaper for pet 1 has a main body portion 2 and a binding portion 40. The main body portion 2 may have a main-body first end 61 which is one end part in the waistline direction W, a main-body second end 62 which is the other end part in the waistline direction W, a main-body third end 63 which is one end part in the cross direction Z, and a main-body fourth end 64 which is the other end part in the cross direction Z. The main-body first end 61, when placed around the waist of pet in the wearing state (see Fig. 4), is covered with the main-body second end 62, and positioned inside (pet side) of the disposable diaper. The main-body second end 62 is opposed to the main-body first end 61, and is disposed on the outside of the disposable diaper in the wearing state. The main-body third end 63 is disposed on the front side of pet in the wearing state. The main-body fourth end 64 is opposed to the main-body third end 63, and is disposed on the back side of pet in the wearing state. The end in the context of this invention is defined as a part that occupies a certain range including the edge. The main-body first end 61 is a part that extends inwardly in the waistline direction W, from a first edge 61E which is one edge in the waistline direction W of the main body portion 2. Likewise, the main-body second end 62 is a part that extends inwardly in the waistline direction W, from a second edge 62E which is the other edge in the waistline direction W of the main body portion 2. The main-body first end and the main-body second end 62 may be regions between the edges in the waistline direction W of the main body portion 2 and the edges in the waistline direction W of the absorbent core 30. The main-body third end 63 is a part that extends inwardly in the cross direction Z from a third edge 63E which is one edge in the cross direction Z of the main body portion 2. Likewise, the main-body fourth end 64 is a part that extends inwardly in the cross direction Z, from a fourth edge 64E which is the other edge in the cross direction Z of the main body portion 2. The main-body third end 63 and the main-body fourth end 64 may be regions between the edges in the cross direction Z of the main body portion 2 and the edges in the cross direction Z of the absorbent core 30.

The main body portion 2 has at least a topsheet 10, a backsheet 20, and an absorbent core 30. The topsheet 10 is arranged to the face to be placed on the pet. The topsheet 10 has liquid permeability that allows body fluid to permeate towards the absorbent core 30. The topsheet 10 may have a center sheet 11 arranged at the center in the cross direction Z so as to cover the absorbent core 30, and side sheets 12 individually covering both side part in the cross direction Z of the center sheet 11. The topsheet 10 may have thereon a printed part. The printed part may form a mark portion 50 described later.

The side sheets 12 may be folded as illustrated in Fig. 3. More specifically, the side sheet 12 on the main-body third end 63 side (left side in Fig. 1) is folded back at the inner edge of the side sheet 12 towards the back face side T2. Meanwhile, the side sheet 12 on the main-body fourth end 64 side (right side in Fig. 1) is folded back at the inner edge of the side sheet 12 towards the top face side T1, and the thus folded part is again folded back towards the top face side T1. A side elastic member 13 while being stretched in the waistline direction W is arranged in between folded parts of the side sheets 12. The side stretchable member 13 may form leakproof gathers that rise up towards the pet. The main-body third end 63 side may be positioned on the front side of pet in the wearing state, meanwhile the main-body fourth end 64 side may be positioned on the back side of pet in the wearing state. The side sheet on one end side in the cross direction Z and the side sheet on the other end side in the cross direction Z, although being asymmetric in this embodiment, may be symmetric in a modified example.

The absorbent core 30 is arranged between the topsheet 10 and the backsheet 20. The absorbent core 30 is made of a stack of pulp or other absorption materials. A core wrap 32 that covers the absorbent core 30 may be provided. In the absorbent core 30, both edges in the waistline direction W of the main body portion 2 is longer than the length in the waistline direction W of the main body portion 2. The absorbent core 30 is arranged at the center in the waistline direction W of the main body portion 2, but is not arranged outside in the waistline direction W of the main body portion 2. The absorbent core 30 may be arranged, in the cross direction Z, inside of both edges of the main body portion 2. That is, the length in the cross direction Z of the absorbent core 30 may be shorter than the length in the cross direction Z of the main body portion 2. The absorbent core 30 may be arranged at the center in the cross direction Z of the main body portion 2, but may not be necessarily arranged outside in the cross direction Z of the main body portion 2. The absorbent core 30 and the core wrap may have a printed part arranged thereto. The printed part may form the mark portion 50.

The backsheet 20 may have a non-liquid permeable back film 21 and a nonwoven back fabric 22 arranged on the outer side of the back film 21. In a modified example, the backsheet may have a non-liquid permeable back film and a nonwoven back fabric arranged on the back side of the back film 21. The back film 21 may have, on a face thereof on the top face side T1, an indicator that causes a coloring reaction upon contact with body fluid, and a printed part, meanwhile the back film 21 may have, on a face thereof on the back face side T2, a printed part. At least one of the indicator or the printed part may form the mark portion 50. That is, the length in the cross direction Z of the back film 21 may be shorter than the length in the cross direction Z of the nonwoven back fabric 22. That is, the nonwoven back fabric 22 may extend on both sides in the cross direction Z out from the back film 21. In an embodiment where the printed part is arranged entirely over the back film 21, an area where the back film 21 is arranged may form a printed area R1, and a non-printed area R2 free of printing may be arranged on both sides in the cross direction Z of the back film 21.

The main body portion 2 may have, on both ends thereof in the cross direction Z, waistline gathers stretchable in the waistline direction W. The waistline gathers reside outside in the cross direction Z of the absorbent core 30, and may be composed of the topsheet 10, the backsheet 20, and a waistline stretchable member 25. The waistline stretchable member 25 may be arranged between the topsheet 10 and the backsheet 20, and may be fixed, while being stretched in the waistline direction W, to the topsheet 10 and the backsheet 20.

The binding portion 40 binds, in the wearing state, opposing parts of the main body portion 2. More specifically, the binding portion 40 is a binding means that binds an outer face 2Q, which is a face on the backsheet side of the main body portion 2, to an inner face 2P, which is a face on the topsheet 10 side of the main body portion 2. The binding portion 40 in this embodiment is a mechanical fastener arranged on the outer face of the main body portion 2. The binding portion 40 is made so as to be bound on the topsheet 10 of the main body portion 2. The binding portion 40 is arranged in the main-body first end 61. When the disposable diaper for pet 1 is placed, the disposable diaper is positioned so that the main-body second end 62 covers the binding portion 40, thereby the binding portion 40 is bound on the topsheet 10, suitably keeping the wearing state of the disposable diaper for pet. In a modified example, the binding portion may be arranged on an inner face of the main body portion 2, and is made engageable with the backsheet of the main body portion 2.

Next, the mark portion 50 will be explained. The mark portion 50 indicates the size in the waistline direction W of the disposable diaper for pet 1. A plurality of the mark portions 50 is arranged with an interval therebetween in the waistline direction. The mark portions 50 in the first embodiment include a first mark portion 51 that resides on the first edge 61E side of the binding portion 40, a second mark portion 52 that resides on the second edge 62E side of the binding portion 40, and a third mark portion 53 that resides on the second edge 62E side of the second mark portion 52. A plurality of mark portions 50 may be arranged in the waistline direction W while placing the binding portion 40 in between, or only on the second edge 62E side of the binding portion 40. The plurality of mark portions may be arranged in the waistline direction at an interval of 10 cm or smaller, which is preferably 7 cm or smaller, and more preferably 5 cm or smaller.

A positional relation between an arbitrary point of the main body portion 2 and the mark portions 50 can vary, depending on mode or wrapping of the disposable diaper around the waist of pet. In an exemplary case where the second edge 62E in the main-body second end 62 falls on the first mark portion 51 in the wearing state, the main-body second end 62 cannot engage with the binding portion 40, so that the user can find that the size of disposable diaper is improper. In an exemplary case where the second edge 62E falls on the second mark portion 52 or the third mark portion 53 in the wearing state, the user can find that the size of disposable diaper is proper. In an alternative case where the second edge 62E falls on the second mark portion 52 in the wearing state, although the second edge 62E regularly falls on the third mark portion 53, the user can find that the waist size has changed and the pet has grown.

With the mark portions 50 arranged with an interval therebetween in the waistline direction., the user can recognize the degree of growth such as increase of the waist size or arrival at the upper limit of suitable size, referring to a positional relation between an arbitrary point of the disposable diaper and the mark portions 50 in the wearing state. The user can therefore choose a suitable size, referring to the mark portions 50. Even if the disposable diaper of improper size has been placed, the user can find that the size is improper referring to the mark portions 50, and can make a new choice of proper size. Some pet causes seasonal changes of hair condition due to self-grooming or depilation, and changes size in the waistline direction W. The user can find the size in the waistline direction W of pet referring to the mark portions 50, can recognize the seasonal changes of size, and can properly place the disposable diaper.

The plurality of mark portions 50 may have same design or different designs. Different designs can make it easier for the user to discriminate the individual mark portions 50. In an embodiment where the mark portion 50 indicates the size of disposable diaper, such mark portion 50 may be accompanied by a corresponding size display. More specifically, the corresponding size display may be given in the mark portion 50 or around the mark portion 50, or may tint the mark portion 50 with a color same as any of package colors for the individual sizes. Such action successfully correlates the mark portion 50 with the size of disposable diaper. Note that different designs in the context of the present invention encompasses not only different pictures or patterns, but also different colors (hues and depths).

The plurality of mark portions 50 may be arranged also with an interval therebetween, in the cross direction Z. The mark portions 50 include fourth mark portions 54 arranged in the cross direction Z with a space in between. The cross direction Z of the disposable diaper for pet, intended to be placed on a pet so as to wrap the waist, extends from the crotch side to a side apart from the crotch. With the plurality of mark portions 50 arranged in the cross direction Z, the user can recognize changes of the waist size in the direction from the crotch side to the side apart from the crotch. For example, the user can recognize, from bulge or dent of the belly, not only the growth process but also changes in physical condition. With the plurality of mark portions 50 thus arranged in the cross direction Z, the user can easily recognize the mark portion 50 from any directions (for example, when viewed from the front side, or from the back side). In a modified example, at least any of the first mark portions 51 to the third mark portions 53, the number of which is two or more, may be arranged also in the cross direction Z with a space in between.

The fourth mark portion 54 may be arranged near an outside part in the cross direction Z of the absorbent core 30, and may point a position of the absorbent core 30. As illustrated in Fig. 4, the disposable diaper for pet 1 is placed by aligning the waistline direction W of the main body portion 2 conforming to the waistline of pet, and by wrapping the disposable diaper for pet 1 around the waist of pet. The absorbent core 30 in this case is not arranged over the entire range in the waistline direction W of the main body portion 2, and is not arranged on both sides in the waistline direction W of the main body portion 2. For example, if the disposable diaper is placed so that an outside part in the waistline direction W of the main body portion 2 (a part not having the absorbent core) is opposed to the excretory opening of pet, the body fluid cannot be absorbed and will leak. In contrast, by aligning the absorbent core 30, referring to the mark portion 50, to an adequate place where excretions can be absorbed, the excretions can now be suppressed from leaking.

The binding portion 40 may be arranged on a face on the back face side of the main-body first end 61, and the mark portion 50 may be arranged on the face on the back face side of the main-body first end 61. When the binding portion 40 arranged on a face on the back face side of the main-body first end 61 is bound with the main-body second end 62, the main-body second end 62 will reside outside of the main-body first end 61. The user, in the process of placement, visually recognizes the second edge 62E of the main-body second end 62 from the outer face (back face) side. In this process, the user can recognize the waist size referring to the positional relation between a position of the second edge 62E and the mark portion 50, and can confirm suitability of size or can enjoy the growth process.

In another embodiment, the binding portion 40 may be arranged on a face on the back face side of the main-body first end 61, and the mark portion 50 may be arranged so as to be visually recognizable from the top face side of the main-body second end 62. When the binding portion 40 arranged on the face on the back face side of the main-body first end 61 is bound with the main-body second end 62, the main-body second end 62 will reside outside of the main-body first end 61, and the back face of the main-body first end 61 and the top face of the main-body second end 62 come into contact. The user can visually recognize, in the process of placement, the binding portion 40 and the mark portion 50 that are brought into contact. The user can recognize the waist size referring to the positional relation between the mark portions 50 of the main-body second end 62 and the binding portion 40, and can confirm suitability of size or can enjoy the growth process.

The first mark portion 51, the second mark portion 52, the third mark portion 53, and the fourth mark portion 54 in this embodiment are formed so as to be visually recognizable from the backsheet side (back face side) of the disposable diaper for pet 1. The backsheet side of the disposable diaper for pet, in the wearing state, appears on the outer side and is visually recognizable after placing the disposable diaper to the pet, enabling the user to recognize the mark portion 50 not only during the process of placement, but also after placement. The mark portion 50 that is visually recognizable from the backsheet side (back face side) of the disposable diaper for pet 1 may be a printed part arranged on the backsheet 20, or may be an indicator arranged on the backsheet 20 or the core wrap 32.

The back film 21 of this embodiment has the printed part arranged over the entire range of the outer face. The printed part may form the mark portion 50, as well as the decoration portion. As illustrated in Figs. 2 and 3, the back film in this embodiment has, on a face on the back face side thereof, a printed part that forms the second mark portion 52, and a printed part that forms a decoration portion 70. The decoration portion 70 is a part having a design other than the mark portion, and may contain not only picture or pattern, but also background color solely represented by color. Note that the decoration portion 70 is not illustrated in Fig. 4.

In another embodiment, the binding portion 40 may be arranged on a face on the top face side of the main-body first end 61, and the mark portions 50 may be arranged so as to be visually recognizable from the back face side of the main-body second end 62. When the binding portion 40 arranged on a face on the top face side of the main-body first end 61 is bound with the main-body second end 62, the main-body first end 61 will reside outside of the main-body second end 62. The user in the process of placement recognizes the outer edge of the main-body first end 61 from the outer face (back face) side. The user in this process can recognize the waist size referring to the positional relation between a position of the first edge 61E of the main-body first end 61 and the mark portions 50, and can confirm suitability of size or can enjoy the growth process.

The mark portion 50 may be made visually recognizable from the topsheet side (top face side) of the disposable diaper for pet 1. The topsheet side of the disposable diaper for pet represents the side faced to the pet upon placement. With the first mark portion 51, the user can easily recognize the mark portions 50 when placing the first mark portion 51 on the body.

The mark portion that is visually recognizable from the topsheet side (top face side) of the disposable diaper for pet 1 may be a printed part arranged on the topsheet 10; may be a printed part arranged on the absorbent core 30 or the core wrap 32; or may be a printed part or an indicator arranged on the backsheet 20, formed so as to be visually recognizable through a low-grammage part of the absorbent core 30. The low-grammage part is a part where the absorbing material has a grammage smaller than the surrounding area, or has zero grammage.

Now "visually recognizable" in the context of this invention means that a subject with healthy vision in both eyes (20/20 vision or higher) can recognize an object from approximately 30 to 50 cm away, in a bright (approximately 500 to 750 lx) room with daylight illumination (color temperature ranging from 4600 to 5400 K). The mark portion 50 may have at least one of the first mark portion 51 or the second mark portion 52.

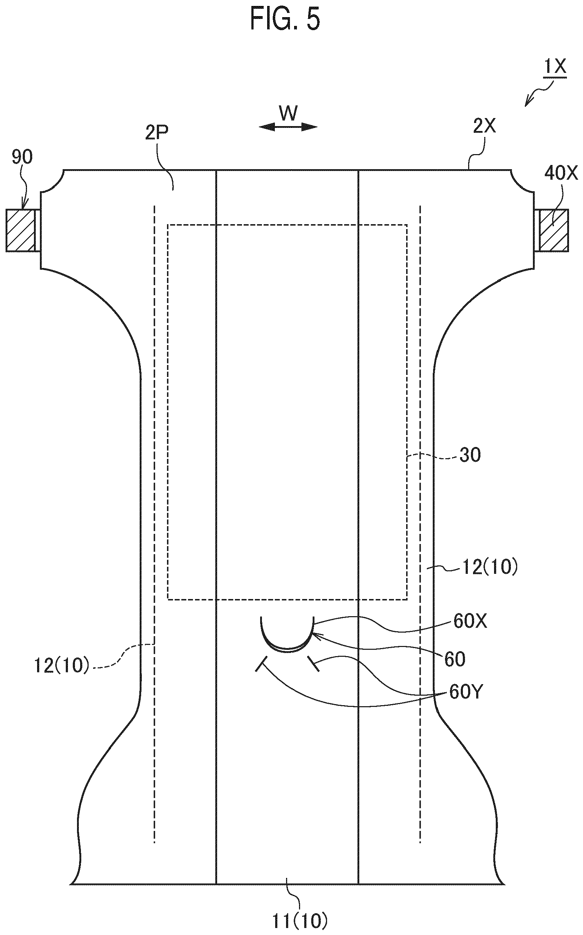

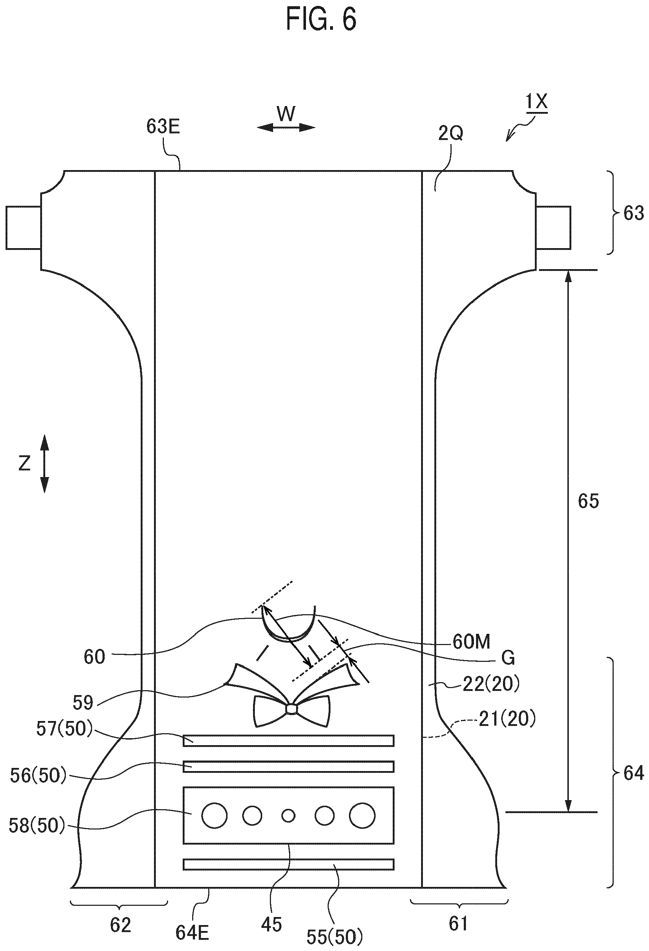

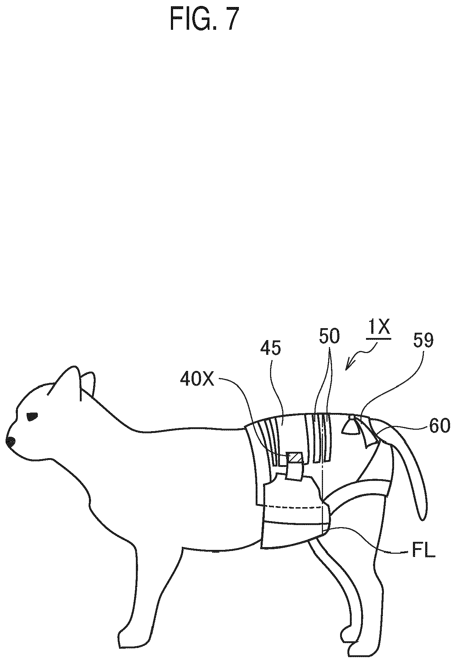

Next, a disposable diaper for pet 1X of the second embodiment will be explained. The disposable diaper for pet of the second embodiment is a disposable diaper for cat. This, however, may be a disposable diaper for dog in a modified example. In the descriptions below in the second embodiment, all structures similar to those in the first embodiment will be given same reference sings, so as to avoid redundant explanation. Fig. 5 is a plan view illustrating the disposable diaper for pet of the second embodiment, viewed from the skin contact side. Fig. 6 is a plan view illustrating the disposable diaper for pet 1X of the second embodiment, viewed from the non-skin contact side. Fig. 7 is a drawing schematically illustrating a wearing state of the disposable diaper for pet 1X of the second embodiment.

The disposable diaper for pet 1X has a main body portion 2X and a binding portion 40X. The disposable diaper for pet 1X of the second embodiment is placed so as to cover the pet from the belly through the crotch to the back. Hence, the disposable diaper for pet 1X of the second embodiment has a length in the cross direction Z, longer than that in the disposable diaper for pet of the first embodiment. The main body portion 2X has the topsheet 10, the backsheet 20, and the absorbent core 30. The absorbent core 30 is biased in the cross direction Z towards the main-body third end 63 that resides on one end side in the cross direction Z of the main body portion 2X. The absorbent core 30 is arranged only on the main-body third end 63 side of a tail hole 60 described later. The length in the cross direction Z of the main body portion 2X is longer than the length in the waistline direction W of the main body portion 2X.

The binding portion 40X extends out from both ends in the waistline direction W of the main body portion 2X, at the main-body third end 63 which is one end in the cross direction Z of the main body portion 2X. In more detail, the binding portion 40X is arranged to the fastening tape 90 that extends, in the main-body third end 63, out from both ends in the waistline direction W of the main body portion 2X. The binding portion 40X is arranged on the inner face 2P of the main body portion 2X, so as to be bindable with a target part 45 arranged on the outer face 2Q of the main body portion 2X. In one modified example, the main body portion may be formed without the target part, and the binding portion 40X may be formed so as to be bindable with the backsheet on the outer face 2Q side of the main body portion 2. In a modified example, the binding portion 40X may extend, in the main-body fourth end 64, out from both ends in the waistline direction W of the main body portion 2X.

When the disposable diaper for pet 1X is placed, the main-body third end 63 (end on the side where the binding portion 40X is arranged) is placed on the belly of pet. In this process, the other end in the cross direction Z of the main body portion 2X is passed between the legs of pet, and left behind the pet. The center in the cross direction Z of the main body portion 2X is then placed on the excretory opening of pet, and the main-body fourth end 64 is placed so as to cover the hip and back of pet. The binding portion 40X is then pulled up towards the back of pet, and attached to the outer face of the target part 45 in the main-body fourth end 64 that reside on the dorsal side. Hence, the disposable diaper for pet 1X may be placed as illustrated in Fig 7, so as to cover the belly, back and crotch of pet. That is, the disposable diaper for pet 1X is placed so as to cover the pet from the belly through the crotch to the back.

The disposable diaper for pet 1X may have formed therein the tail hole 60 through which the tail can be inserted. The tail hole 60 has a through-hole 60X through which the tail of pet can be inserted, and cut parts 60Y that can enlarge the through-hole. When the disposable diaper with the tail hole 60 is placed, the tail of pet may be inserted in the through-hole 60X of the tail hole 60, in the process of covering the hip and back of pet with the other end in the cross direction Z of the main body portion 2X in a wearing state. The through-hole 60X may be a half-arc cut. The cut part 60Y may typically be a perforation along which the topsheet 10 and the backsheet 20 are tearable, making the size of through-hole 60X variable depending on the species and growth process of pet. The main-body fourth end 64 in the second embodiment may be a region that ranges between the edge in the cross direction Z of the main body portion 2 and the edge in the cross direction Z of the absorbent core 30; or may be a region that ranges between the edge in the cross direction Z of the main body portion 2 and the tail hole 60.

As illustrated in Fig. 6, the main body portion 2X may have a pair of leg surrounding openings 65 given by the outer edges in the waistline direction W of the main body portion 2X, which dent inwardly in the waistline direction W, and placed around the legs of pet. The leg surrounding openings 65 are parts that are arranged across the center in the cross direction Z of the disposable diaper, and dent inwardly in the waistline direction W.

As illustrated in Fig. 6, a plurality of the mark portions 50 is arranged with an interval therebetween in the cross direction Z The mark portions 50 in the second embodiment have a fifth mark portion 55 arranged in the main-body fourth end 64 and positioned on the fourth edge 64E side of the target part 45, a sixth mark portion 56 positioned on the third edge 63E side of the target part 45, and a seventh mark portion 57 positioned on the third edge 63E side of the sixth mark portion 56. A plurality of mark portions 50 may be arranged in the cross direction Z while placing the target part 45 in between, or only on the third edge 63E side of the target part 45.

In an embodiment where the disposable diaper is placed so as to cover the pet from the belly through the crotch to the back, the positional relation between an arbitrary point of the main body portion and the mark portions 50 will vary depending on the longitudinal length of the body of pet. In an exemplary case where the third edge of the main-body third end 63 falls on the fifth mark portion 55 in the wearing state, the main-body third end 63 cannot engage with the binding portion 40X, so that the user can find that the size of disposable diaper is improper. Meanwhile, in an exemplary case where the third edge 63E falls on the sixth mark portion 56 or the seventh mark portion 57 in the wearing state, the user can find that the size of disposable diaper is proper. In an alternative case where the third edge falls on the sixth mark portion 56 in the wearing state, although regularly falls on the seventh mark portion 57, the user can find that the waist size has changed and the pet has grown.

With the mark portions 50 arranged with an interval therebetween in the cross direction Z, the user can recognize the degree of growth, such as increase of the longitudinal size or arrival at the upper limit of suitable size, referring to a positional relation between an arbitrary point of the disposable diaper and the mark portions 50 in the wearing state. The user can therefore choose a suitable size, referring to the mark portions 50. Even if the disposable diaper of improper size has been placed, the user can find that the size is improper referring to the mark portions 50, and can make a new choice of proper size. A typical human infant disposable diaper that extends for coverage from the belly through the crotch to the back of a child is usually placed so as to mate one end and the other end in the cross direction, while allowing some vertical shift of the placement position. In contrast, the disposable diaper for pet having the tail hole cannot easily shift the placement position vertically, occasionally making the one end and the other end in the cross direction unmatched. Placement by the user so as to match the one end and the other end in the cross direction would inadequately tense or slacken the disposable diaper on the ventral side. The disposable diaper can, however, according to this embodiment, remain at a proper position, on the basis of positional relation of the mark portions in a proper wearing state on the pet.

At least one mark portion 50 out of the plurality of mark portions 50 may be arranged in an area that falls between the pair of leg holes 65. When using the disposable diaper to be extended for coverage from the belly through the crotch to the back of a pet, the binding portion 40X is preferably bound along a line (FL in Fig. 7) which extends from the base of the legs on the ventral side to the back. With the binding portion 40X bound along the line which extends from the base of legs on the ventral side to the back, fitting quality around the legs will improve, and thereby leakage or displacement may be suppressed. With the mark portions 50 provided in an area that falls between the leg openings, the binding portion 40X may be bound referring to the mark portions 50 in an area around the legs.

A plurality of the mark portions 50 may be arranged also with an interval therebetween in the waistline direction. The mark portions 50 in the second embodiment have eighth mark portions 58 arranged with an interval therebetween in the cross direction Z. The eighth mark portions 58 are arranged in the target part 45. The plurality of the eighth mark portions 58 has different sizes, increasing from the center in the waistline direction W outwards in the waistline direction W. The positional relation between the fastening tape 90 and the eighth mark portions 58 will vary, depending on modes of attachment of the binding portion 40X to the target part. For example, if the smallest eighth mark portion 58 appears close to the fastening tape, the user can recognize that the disposable diaper is still large enough. Meanwhile, if the largest eighth mark portion 58 appears close to the fastening tape, indicating increase of the waist size, the user can recognize that it is the time to upsize the disposable diaper. In a modified example, at least any of the fifth mark portions 55 to the seventh mark portions 57, the number of which is two or more, may be arranged also in the waistline direction W with a space in between.

The mark portions 50 may have a tail mark portion 59 that is formed so as to be visually recognizable from the backsheet side of the disposable diaper for pet, and points the tail hole 60. The user will be aware of the presence of the tail hole 60 with the aid of the tail mark portion 59, and can insert the tail through the tail hole 60 to place the disposable diaper. With the tail inserted through the tail hole 60, the user can properly align the direction of the main body portion 2 for placement in the suitable position. The user also can recognize the growth process of pet, through the thickness and length of the tail inserted through the tail hole 60. With the disposable diaper placed on the pet, the user can recognize both of the mark portions 50 and the conditions of the tail inserted through the tail hole 60, and can determine whether or not to enlarge the tail hole using the cut part 60Y

The tail mark portion 59 may be arranged near the tail hole 60. Referring now, for example, to the maximum size 60M of the tail hole 60 in plan view illustrated in Fig. 6, the minimum gap G between the tail hole 60 and the tail mark portion 59 may be equal to or smaller than maximum size 60M. The maximum gap between the tail hole 60 and the tail mark portion 59 may preferably be equal to or smaller than maximum size 60M. Alternatively, the minimum gap between the tail hole 60 and the tail mark portion 59 may be 20 mm. The maximum gap between the tail hole 60 and the tail mark portion 59 may preferably be 20 mm. At least a part of the tail mark portion 59 more preferably overlaps the tail hole. The user will easily recognize both of the tail mark portion and the tail hole at the same time, and will become more aware of the presence of the tail hole with the aid of the tail mark portion.

The tail mark portion 59 may point the direction of placement of the disposable diaper for pet. The direction of placement is specifically exemplified by vertical direction and lateral direction in the process of placement. In a mode where the disposable diaper is placed so as to cover the pet from the belly through the crotch to the back, the user would sometimes fail in correctly discriminating the regions to be placed on the belly and the back, and would accidentally invert the belly side and the back side. Now with the tail mark portion 59 for pointing the direction of placement of the disposable diaper for pet, the direction of placement of the disposable diaper can be pointed during the placement, and thereby the user can place the disposable diaper in a correct direction. Even in case of placement in the wrong direction, the user can visually recognize the tail mark portion 59 after the placement, and can correct the disposable diaper in the right direction.

The tail mark portion 59 that points the direction of placement may point the direction with a design having vertical directionality, or with letter, numeral or symbol having vertical directionality. The disposable diaper placed in the correct direction makes the design or letter directed in the correct direction, making it possible to point the direction of placement of disposable diSper. The tail mark portion 59 in this embodiment has an illustration of ribbon, where the correct direction is given by two loops extending radially from a knot positioned upside, and the trails falling down from the knot positioned downside. The correct direction of placement is thus pointed by the illustration of ribbon.

In another embodiment of the second embodiment, the binding portion 40X may be arranged on a face on the back face side of the main-body third end 63, and the mark portions 50 may be arranged on a face on the back face side of the main-body third end 63. When the binding portion arranged on a face on the back face side of the main-body third end 63 is bound with the main-body fourth end 64, the main-body fourth end 64 will reside outside of the main-body third end 63. The user, in the process of placement, visually recognizes the edge of the main-body fourth end 64 from the outer face (back face) side. In this process, the user can recognize the waist size referring to the positional relation between a position of the edge of the main-body fourth end 64 and the mark portions 50, and can confirm suitability of size or can enjoy the growth process.

In another embodiment, the binding portion 40X may be arranged on a face on the back face side of the main-body third end 63, and the mark portions 50 may be arranged so as to be visually recognizable from the top face side of the main-body fourth end 64. When the binding portion arranged on a face on the back face side of the main-body third end 63 is bound with the main-body fourth end 64, the main-body fourth end 64 will reside outside of the main-body third end 63, and the back face of the main-body third end 63 and the top face of the main-body fourth end 64 come into contact. The user can visually recognize, in the process of placement, the binding portion and the mark portion 50 that are brought into contact. The user can recognize the waist size referring to the positional relation between the mark portions 50 of the main-body fourth end 64 and the binding portion, and can confirm suitability of size or can enjoy the growth process.

Next, an exemplary method for manufacturing the thus designed disposable diaper for pet will be explained. The method for manufacturing the disposable diaper for pet may include: a sheet feeding step of feeding a continuous sheet having sheet components arrayed therein; a marking step of giving a mark portion by printing on the continuous sheet to provide the mark portion 50; and an absorbent core laminating step of laminating the absorbent core 30 on the continuous sheet having the mark portion 50.

The sheet feeding step, feeding the continuous sheet having sheet components arrayed therein, takes part in feeding of the continuous sheet in which sheet components, composing any one of the topsheet 10, backsheet and core wrap, are arrayed. The marking step takes part in giving the mark portion 50 on the continuous sheet. The mark portion 50 may be given by printing, or by embossing. The core laminating step takes part in detection of the mark portion 50 on the continuous sheet. The mark portion 50 may be detected by detecting a position thereof using an image capturing means such as camera. The core laminating step takes part in adjustment of a position of lamination of the absorbent core 30, referring to the position of the mark portion 50. Adjustment of the position of lamination of the absorbent core 30 means adjustment of the positional relation between the absorbent core 30 and the continuous sheet. More specifically, the positional relation between the continuous sheet and the absorbent core 30 is adjustable by controlling the rate of feeding of the continuous sheet. Alternatively, an adjustment mechanism may be arranged adjacent to the sides, in a cross direction orthogonal to the feeding direction, of the continuous sheet, and a position in the orthogonal direction of the continuous sheet may be adjusted using such adjustment mechanism. With such method for manufacturing, the positional relation between the mark portion 50 and the absorbent core 30 may be kept constant. Hence, the disposable diaper when placed referring to the mark portion 50 will have the absorbent core 30 whose placement position is prevented from being misaligned one by one. With the mark portion 50 suppressed from shifting over the whole range of the disposable diaper, it now becomes possible to improve accuracy of alignment and accuracy of pointing of the degree of growth, with the aid of the mark portions 50.

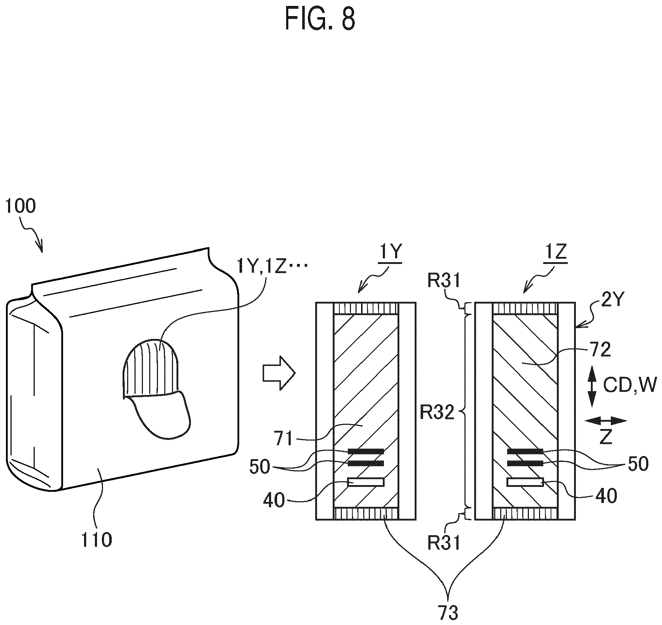

Next, a package 100 having a plurality of disposable diapers for pet enclosed therein will be explained, referring to Fig. 8. The package 100 contains a plurality of disposable diapers for pet 1Y and 1Z, and a wrapper 110 for enclosing the plurality of disposable diapers for pet 1Y and 1Z. The plurality of disposable diapers for pet includes the first disposable diaper 1Y having the first decoration portion 71 printed on the backsheet 20, and the second disposable diaper 1Z having the second decoration portion 72 designed differently from the first decoration portion 71 printed on the backsheet 20. The first decoration portion 71 and the second decoration portion 72 may only have designs different from each other, and may be different from the first decoration portion 71 and the second decoration portion 72 in the second embodiment. In this embodiment, the first decoration portion or the second decoration portion is printed on the back film of the backsheet. With such first disposable diaper having the first decoration portion and the mark portions 50, and the second disposable diaper having the second decoration portion 72 and the mark portions 50, this embodiment will have enhanced decorativeness, thereby can attract attention of the user, and can improve recognizability of the mark portions 50. The first decoration portion and the second decoration portion are parts having a design other the mark portion 50, and may contain not only picture or pattern, but also background color solely represented by color.

The backsheet 20 is laid over the entire range of a main body portion 2Y in a continuous direction CD which is at least either the waistline direction W or the cross direction Z. The backsheet 20 in this embodiment is laid continuously in the waistline direction W, and the continuous direction CD coincides with the waistline direction W. The backsheet 20, having the first decoration portion 71 and the second decoration portion 72, is arranged over the entire range of the main body portion 2Y in the continuous direction, and can make the decoration portions distinctive. The first decoration portion, the second decoration portion and the mark portions 50 are arranged likewise on the backsheet, wherein the first decoration portion and the second decoration portion attract attention of the user, to thereby enhance recognizability of the mark portions 50.

The mark portions 50 of the first disposable diaper and the mark portions 50 of the second disposable diaper may have the same design. The user can easily recognize the presence and function of the mark portions 50. Alternatively, the mark portions 50 of the first disposable diaper and the mark portions 50 of the second disposable diaper may have different designs. Difference in designs between the mark portions 50 of the first disposable diaper and the mark portions 50 of the second disposable diaper can increase design variations, and can enhance the decoration effect. The disposable diapers enclosed in the wrapper may only be at least the first disposable diaper and the second disposable diaper, or may include a third disposable diaper having a third decoration portion different from the first decoration portion and the second decoration portion, or may include multiple types of disposable diaper.

The backsheet may have outer regions R31 that extend inwards from the outer edges in the continuous direction CD of the backsheet, and an inner region R32 that resides inside, in the continuous direction, of the outer regions R31. The first decoration portion 71 and the second decoration portion 72 may be arranged in the inner region R32. The outer regions R31 of the first disposable diaper and the outer region R31 of the second disposable diaper may have common decoration portions 73 with a unified design printed therein. The common decoration portions 73 may be different from the first decoration portion 71 and the second decoration portion 72. That is, the first disposable diaper 1Y has the first decoration portion 71 in the inner region R32, and the common decoration portions 73 in the outer regions R31. Meanwhile, the second disposable diaper 1Q has the second decoration portion 72 in the inner region R32, and the common decoration portions 73 in the outer regions R31. Since the outer regions R31 and the inner region R32 are differently designed in both of the first disposable diaper and the second disposable diaper, so that the user can more easily recognize the position in the main body portion 2Y with the aid of the designs. When recognizing the position of the mark portions 50, the user can easily find the position over the entire range of the main body portion 2Y, and can more properly understand the degree of growth of pet, referring to the mark portions 50. That is, the differently designed first decoration portion 71 and the second decoration portion 72 can enhance the decorativeness, meanwhile the common decoration portions 73 can make the user more easily recognize the position of components (main body portion 2Y and mark portions 50).

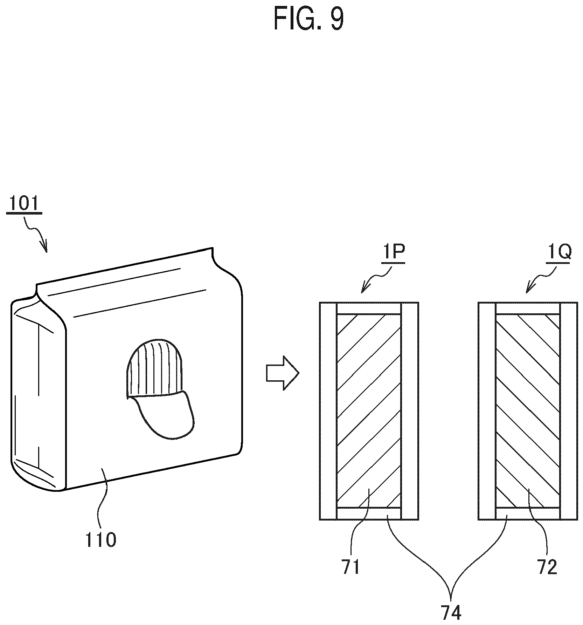

Next, a package 101 of a modified example will be explained, referring to Fig. 9. The package 101 contains a plurality of disposable diapers for pet 1P and 1Q, and a wrapper 110 for enclosing the plurality of disposable diapers for pet 1P and 1Q. In the explanation of the package 101 of the disposable diapers for pet, all structures same as those in the aforementioned package 100 of the disposable diapers for pet will be given same reference signs in order to skip the description. The plurality of disposable diapers for pet in the package 101 includes at least the first disposable diaper 1P and the second disposable diaper 1Q. The outer region R31 of the first disposable diaper 1P and the outer region R31 of the second disposable diaper 1Q have, arranged therein, a non-printed part 74 free of printing.

The first disposable diaper 1P has the first decoration portion 71 in the inner region R32, and the non-printed part 74 in the outer region R31. Meanwhile, the second disposable diaper 1Z has the second decoration portion 72 in the inner region R32, and the non-printed part 74 in the outer regions R31. With the non-printed part 74 arranged
in the outer regions R31, and with the decoration portion arranged in the inner region R32, the user will easily recognize the position in the main body portion, referring to the design, or presence or absence of such design. When recognizing the position of the mark portions 50, the user can easily find the position over the entire range of the main body portion, and can more properly understand the degree of growth of pet, referring to the mark portions 50. The differently designed first decoration portion and the second decoration portion can enhance the decorativeness, meanwhile the common non-printed part can make the user more easily recognize the position of components (main body portion and mark portions 50) over the entire range of the disposable diaper.

The embodiments of the present invention have been described above in detail, it is apparent for persons who have ordinary skill in the art that the embodiments described in this specification do not limit the scope of the present invention. Changes and modifications may apply to the embodiments of the present invention without departing from the spirit and scope of the present invention that are defined by the description of the appended claims. The description of the present specification, therefore, has been understood to be illustrative and is in no way intended to limit the scope of the invention.

### [INDUSTRIAL APPLICABILITY]

According to the present invention, it is possible to provide a disposable diaper for pet, capable of making the user easily recognize the degree of growth of its pet upon placement of the disposable diaper.
1 disposable diaper for pet,
2:Main body portion
10:Topsheet
20:Backsheet
30: absorbent core
40:Binding portion
50:Mark portions
59: tail mark portion
61: main-body first end
62: main-body second end
63: main-body third end
64: main-body fourth end
65: leg surrounding opening
71: first decoration portion
72: second decoration portion
73: common decoration par

## Claims

1. A disposable diaper for pet (1), comprising:
a waistline direction (W) disposed along a waistline direction of a pet;
a cross direction (Z) orthogonal to the waistline direction (W);
a main body portion (2) having a topsheet (10), a backsheet (20), and an absorbent core (30) arranged between the topsheet (10) and the backsheet (20);
a binding portion (40, 40X) that extends outwards in the waistline direction (W) from the main body portion (2), in a main-body third end (63) that resides on one end side in the cross direction (Z) of the main body portion (2); and
a target part (45) with which the binding portion (40, 40X) is bindable, and
the disposable diaper for pet (1) being intended to be placed to cover the pet from a belly through a crotch to a back of the pet, **characterized in that** the disposable diaper for pet (1) has a plurality of mark portions (50) having mark portions (55, 56, 57) in the cross direction (Z) and mark portions (58) in the waistline direction (W),
the mark portions (55, 56, 57) in the cross direction (Z) are arranged with an interval therebetween in the cross direction (Z),
the mark portions (58) in the waistline direction (W) are arranged with an interval therebetween in the waistline direction (W), and
the mark portions (58) in the waistline direction (W) are arranged in the target part (45).

2. The disposable diaper for pet according to claim 1, wherein the main body portion (2) has a pair of leg surrounding openings (65) to be arranged around legs of pet, composed of outer edges in the waistline direction (W) of the main body portion (2) recessed inwards in the waistline direction (W), and
at least one mark portion (50), out from the plurality of mark portions (50), is arranged in a region that falls between the pair of leg surrounding openings (65).

3. The disposable diaper for pet according to claim 1 or 2, further comprising a tail hole (60) that has a through-hole (60X) through which the tail of pet can be inserted, and a cut part (60Y) that can enlarge the through-hole (60X), and
the mark portion (50) having a tail mark portion (59) that is formed so as to be visually recognizable from the backsheet side of the disposable diaper for pet (1), and indicates the tail hole (60).

4. A package (100, 101) of a disposable diaper for pet, comprising a plurality of disposable diapers (1Y, 1Z, 1P, 1Q) for pet described in any one of claims 1 to 9, and a wrapper (110, 111) that encloses the plurality of disposable diapers (1Y, 1Z, 1P, 1Q) for pet, wherein
the disposable diapers (1Y, 1Z, 1P, 1Q) for pet include a first disposable diaper (1Y, 1P) having a first decoration portion (71) and the mark portion (50) printed on the backsheet (20), and a second disposable diaper (1Z, 1Q) having a second decoration portion (72) designed differently from the first decoration portion (71), and the mark portion (50) printed on the backsheet (20), and
the backsheet (20) is arranged over the entire range of the main body portion (2) in a continuous direction (CD) that contains at least either the waistline direction (W) or the cross direction (Z).

5. The package according to claim 4, wherein the backsheet (20) has an outer region (R31) that extends inwards from an outer edge in the continuous direction (CD) of the backsheet (20), and an inner region (R32) that resides inside, in the continuous direction (CD), of the outer region (R31),
the first decoration portion (71) and the second decoration portion (72) are arranged in the inner region (R32),
the first disposable diaper (1Y) and the second disposable diaper (1Z) have a common decoration portion (73) having a unified design, printed in the outer regions (R31) of the first disposable diaper (1Y) and the second disposable diaper, and
the common decoration portion is designed differently from the first decoration portion (71) and the second decoration portion (72).

6. The package according to claim 4, wherein the backsheet (20) has an outer region (R31) that extends inwards from the outer edge in the continuous direction (CD) of the backsheet (20), and an inner region (R32) that resides inside, in the continuous direction (CD), of the outer region (R31),
the first decoration portion (71) and the second decoration portion (72) are arranged in the inner region (R32), and
each of the first disposable diaper (1P) and the second disposable diaper (1Q) has, in the outer regions (R31) of the first disposable diaper (1P) and the second disposable diaper (1Q), a non-printed region free of printing.
